# EUROPEAN PATENT APPLICATION

(11) **EP 4 248 851 A1**
(43) Date of publication of application: **27.09.2023**
(21) Application number: 21894088.0
(22) Date of filing: 07.10.2021
(51) Int. Cl.: A61B 5/022, A61B 50/30

(54) **SINGLE-USE PROTECTIVE SLEEVE INTENDED TO HOUSE A SPHYGMOMANOMETER CUFF**

(30) Priority: 19.11.2020 ES 202032506 U
(71) Applicant: Universidad de Almería, 04120 la Cañada de San Urbano (ALMERÍA) (ES)
(72) Inventor: MÁRQUEZ HERNÁNDEZ, Verónica V., 04120 La Cañada de San Urbano (ALMERÍA) (ES); GUTIÉRREZ PUERTAS, Lorena, 04120 La Cañada de San Urbano (ALMERÍA) (ES); AGUILERA MANRIQUE, Gabriel, 041020 La Cañada de San Urbano (ALMERÍA) (ES); ALCAYDE GARCÍA, Alfredo, 04120 La Cañada de San Urbano (ALMERÍA) (ES); GIL MONTOYA, Francisco, 04120 La Cañada de San Urbano (ALMERÍA) (ES); MANZANO AGUGLIARO, Francisco, 04120 La Cañada de San Urbano (ALMERÍA) (ES); GARRIDO MOLINA, José Miguel, 04120 La Cañada de San Urbano (ALMERÍA) (ES); GARCÍA VIOLA, Alba, 04120 La Cañada de San Urbano (ALMERÍA) (ES); PEÑA FERNÁNDEZ, Ana Araceli, 04120 La Cañada de San Urbano (ALMERÍA) (ES)
(74) Representative: Pons
(86) International application number: PCT/ES2021/070731
(87) International publication number: WO 2022/106735

(57) **Abstract**

The invention relates to a single-use protective cover (1) intended to house a sphygmomanometer cuff and to adapt to the shape thereof, comprising a lower portion provided with an open side allowing the sphygmomanometer cuff to be inserted, wherein said protective sleeve (1) is made from a single piece of a hydrophobic material of SMS type consisting of a non-woven fabric comprising a top layer of spunbond polypropylene, a middle layer of blow-moulded polypropylene and a bottom layer of spunbond polypropylene, said protective sleeve (1) thus acting as a barrier against the risk of infection.

## Description

### OBJECT OF THE INVENTION

The present invention relates to a belt-like single-use protective sleeve intended to house a sphygmomanometer cuff and furthermore intended to adapt to the shape thereof, which is comprised in the field of devices or accessories that protect against the risk of infections in sphygmomanometers cuffs. More particularly, the present invention describes a single-use protective sleeve suitable for housing a sphygmomanometer cuff, wherein said protective sleeve is made from a single piece of a hydrophobic material acting as a barrier against the risk of infections.

### BACKGROUND OF THE INVENTION

Taking blood pressure in clinical practice is one of the most commonly performed techniques for the hemodynamic assessment of patients, either in emergency situations or in routine examinations. Of all the methods used for taking blood pressure, the auscultatory method with the use of a sphygmomanometer is the most accurate, widely used, studied and investigated method.

The sphygmomanometer is an apparatus for the indirect measurement of blood pressure using an armband (called a cuff) which is placed on the arm, a rubber bulb to inflate the armband, and a stethoscope to listen to the pulse.

Traditionally, taking blood pressure with a sphygmomanometer has been considered an innocuous and safe method which provides important information about the patient's state of health. However, these devices are used repeatedly on different patients, without disinfecting them, which prevents guaranteeing safe and quality care, which is essential after the Covid-19 pandemic.

There are various scientific studies that demonstrate that the sphygmomanometer cuff incorporates organic debris and microorganisms from the patient's skin. Therefore, it can constitute a reservoir and route for bacterial transmission with a potential danger of infection for patients; and a health risk for healthcare professionals.

Utility model U201000015 describes a single-use protector for phonendoscope or stethoscope bells and diaphragms, which could constitute a complementary element for the present invention. However, protective devices for sphygmomanometer cuffs are not known in the state of the art.

### DESCRIPTION OF THE INVENTION

The present invention aims to solve some of the problems mentioned in the state of the art. More particularly, the present invention describes a belt-like single-use protective sleeve intended to house a sphygmomanometer cuff and to adapt to the shape thereof, wherein said protective sleeve is made from a single piece of a hydrophobic material acting as a barrier against the risk of infections.

In a preferred embodiment, it is made from a material of SMS type ("Spunbond Meltblown Spunbond fabric") consisting of a non-woven fabric comprising a top layer of spunbond polypropylene, a middle layer of blow-moulded polypropylene and a bottom layer of spunbond polypropylene. Mas preferably, it is made from SMS 35 g.

Likewise, the protective sleeve can also be made from a non-woven fabric of SMMS type ("Spunbond, Meltblown, Meltblown and Spunbond"), SSMMS type ("Spunbond, Spunbond, Meltblown, Meltblown and Spunbond") and even SMMMS type which incorporates 3 meltblown layers.

The protective sleeve can have an open lateral side to insert the sphygmomanometer cuff into the protective sleeve.

Preferably, the protective sleeve further comprises an attachment element in an upper or lower portion of the open side, intended to attach the protective sleeve with the sphygmomanometer cuff housed internally. In a preferred embodiment, said attachment element is a double-sided adhesive.

Likewise, the protective sleeve can be provided with a position marking for the artery which indicates the range of placement both on an outer surface and on an inner surface.

The protective sleeve can be provided with a through hole on a side surface thereof.

The single-use protective sleeve can be made for the different sizes of armbands or cuffs existing on the market (XL, adult, children) and in different colours.

### DESCRIPTION OF THE DRAWINGS

As a complement to the description provided herein, and for the purpose of helping to make the features of the invention more readily understandable, in accordance with a preferred practical exemplary embodiment thereof, said description is accompanied by a set of drawings constituting an integral part of the same, which by way of illustration and not limitation, the following has been represented:
Figure 1 shows a front view of an outer surface of the protective sleeve according to a preferred embodiment of the invention, in which the internal surface comprising openings to leave the Velcro fastening of the cuff uncovered, as well as the position marking for the artery, are illustrated.
Figure 2 shows a front view of an inner surface of the protective sleeve according to a preferred embodiment of the invention, in which the external surface comprising openings to leave the Velcro fastening of the cuff uncovered, as well as the position marking for the artery and the side through hole, are illustrated.
Figure 3 shows a perspective view of a lower portion of the protective sleeve, in which it is shown that the sleeve comprises an open side and an adhesive.
Figure 4 shows a perspective view of an opposite view with respect to Figure 3, in which the open side, the opening, and the Velcro fastening of the cuff are shown.

### PREFERRED EMBODIMENT OF THE INVENTION

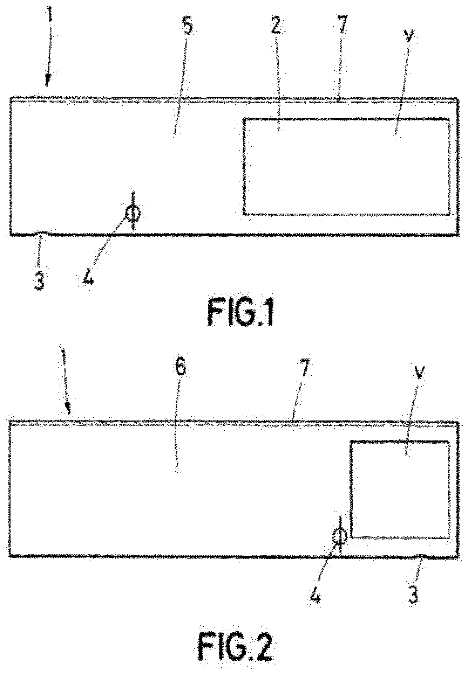

A preferred embodiment of the single-use protective sleeve (1) intended to house a sphygmomanometer cuff and to adapt to the shape thereof is described below with the help of Figures 1-3.

In the preferred embodiment which has been described, the sphygmomanometer cuff intended to be housed in the protective sleeve (1) is of the type comprising a Velcro fastening (V) to close around and to adapt to the anatomy by way of an armband or a brace.

Figure 1 shows a front view of the protective sleeve (1) in which it is shown that the sleeve comprises an internal surface (5) comprising openings (2) intended to allow accessibility to the Velcro fastening (v) of the cuff. The portion having the Velcro fastening (v) of the original cuff is therefore uncovered to ensure the correct placement of the armband or cuff on the user.

In the preferred embodiment which has been described, the protective sleeve (1) is made from a single piece of a hydrophobic material acting as a barrier against the risk of infections, wherein said material is a non-woven fabric of SMS ("Spunbond Meltblown Spunbond fabric") 35 g type comprising a top layer of spunbond polypropylene, a middle layer of blow-moulded polypropylene and a bottom layer of spunbond polypropylene.

Figure 2 shows a view of the external surface (6) which also comprises openings (2) intended to allow accessibility to the Velcro fastening (v) of the cuff.

Likewise, both the internal surface (6) and the external surface (5) are provided with a position marking (4) for the artery which indicates the range of placement.

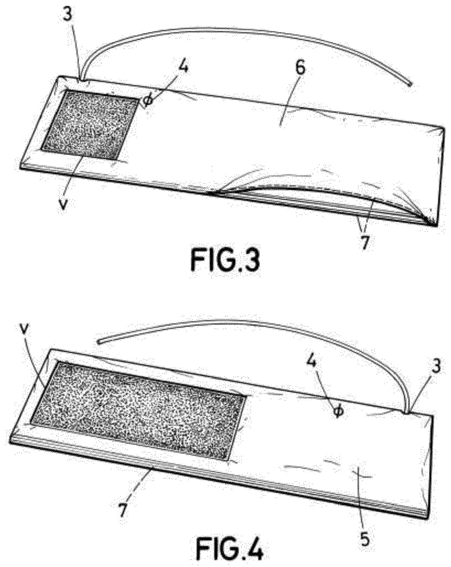

Figure 3 shows a perspective view of a lower portion of the protective sleeve (1) according to the preferred embodiment, in which it is illustrated that the protective sleeve (1) has an open surface and a double-sided adhesive line (7) to allow correct placement on the cuff.

Figure 4 shows a perspective view of the portion opposite that of Figure 3 according to the preferred embodiment of the protective sleeve.

## Claims

1. A single-use protective sleeve (1) intended to house a sphygmomanometer cuff and to adapt to the shape thereof, wherein said protective sleeve (1) is **characterised in that** it is made from a single piece of a hydrophobic material acting as a barrier against the risk of infections.

2. The protective sleeve (1) according to claim 1, wherein the hydrophobic material is a SMS ("Spunbond Meltblown Spunbond fabric") consisting of a non-woven fabric comprising a top layer of spunbond polypropylene, a middle layer of blow-moulded polypropylene and a bottom layer of spunbond polypropylene.

3. The protective sleeve (1) according to claim 1, comprising an opening (2) on an inner surface (5) and on an outer surface (6), wherein said openings (2) are intended to leave the Velcro fastening (v) of the sphygmomanometer cuff uncovered to ensure the correct placement thereof with the protective sleeve (1).

4. The protective sleeve (1) according to claim 1, comprising in a lower portion an open side allowing the sphygmomanometer cuff to be inserted.

5. The protective sleeve (1) according to claim 4, further comprising an attachment element (7) in a lower portion or in an upper portion of the open side intended to attach the protective sleeve (1) with the sphygmomanometer cuff.

6. The protective sleeve (1) according to claim 5, wherein the attachment element (7) is a double-sided adhesive.

7. The protective sleeve (1) according to claim 1, comprising on the inner surface (5) and on the outer surface (6) a position marking (4) for the artery which indicates the range of placement.

8. The protective sleeve (1) according to claim 1, comprising a through hole (3) in a side portion thereof.
